# EUROPEAN PATENT SPECIFICATION

(11) **EP 2 663 634 B1**
(45) Date of publication and mention of the grant of the patent: **15.08.2018**
(21) Application number: 11855320.5
(22) Date of filing: 29.12.2011
(51) Int. Cl.: C12N 1/18, C12N 1/38, C12P 7/06

(54) **NUTRIENT COMPOSITION FOR SACCHAROMYCES CEREVISIAE AND USAGE METHOD THEREOF**
NÄHRSTOFFZUSAMMENSETZUNG FÜR SACCHAROMYCES CEREVISIAE UND VERWENDUNGSVERFAHREN
COMPOSITION NUTRITIVE POUR SACCHAROMYCES CEREVISIAE ET SA MÉTHODE D'UTILISATION

(30) Priority: 10.01.2011 CN 201110008032
(43) Date of publication of application: 20.11.2013
(73) Proprietor: Angel Yeast Co., Ltd., Hubei 443003 (CN)
(72) Inventor: YU, Xuefeng, Yichang Hubei 443003 (CN); LI, Zhihong, Yichang Hubei 443003 (CN); YU, Minghua, Yichang Hubei 443003 (CN); YAO, Juan, Yichang Hubei 443003 (CN); LEI, Jincheng, Yichang Hubei 443003 (CN); XU, Yinhu, Yichang Hubei 443003 (CN); LI, Zhijun, Yichang Hubei 443003 (CN)
(74) Representative: Hryszkiewicz, Danuta
(86) International application number: PCT/CN2011/002216
(87) International publication number: WO 2012/094798

(56) References cited:
- WO-A1-92/20777
- CN-A- 101 768 556
- JP-A- 2006 109 720
- JP-B2- 3 720 780
- KR-B1- 100 429 494
- US-A1- 2008 187 987
- US-A1- 2010 184 196
- KAMATH ET AL: "Optimization of cultural conditions for protease production by a fungal species", INDIAN JOURNAL OF PHARMACEUTICAL SCIENCES , 1 July 2010 (2010-07-01), XP002743113, Retrieved from the Internet: URL:http://www.ijpsonline.com/text.asp?201 0/72/2/161/65017 [retrieved on 2015-08-07]
- LI LI-QUN ET AL.: 'Experimental research on the high gravity fermentation with Faerkai' TH-AADY, JOURNAL OF JILIN INSTITUTE OF CHEMICAL TECHNOLOGY vol. 24, no. 1, 28 February 2007, pages 6 - 9, XP008170666
- DONATELLA BARRA ET AL.: 'A Tetrameric Iron Superoxide Dismutase from the Eucaryote Tetrahymena pyriformis' THE JOURNAL OF BIOLOGICAL CHEMISTRY vol. 265, no. 29, 15 October 1990, pages 17680 - 17687, XP008163215
- VOIGT GLOBAL DISTRIBUTION INC: "Difco Yeast Extract, Difco Yeast Nitrogen Base, Difco Yeast Nitrogen Base w/o Amino Acids, Difco Yeast Nitrogen Base w/0 Amino Acids and Ammonium Sulfate", INTERNET CITATION, [Online] XP002448840, Retrieved from the Internet: URL:http://www.voigtglobal.com/Anonymous/D IFCO_Yeast_Agar_Formulations.pdf> [retrieved on 2007-08-29]

## Description

The invention relates to a nutrient composition for *Saccharomyces cerevisiae* and a usage method thereof.

Nutrients are essential for the life activities of *Saccharomyces cerevisiae,* without it, *Saccharomyces cerevisiae* will come to an end. Nutritional deficiency or insufficient nutrients would cause *Saccharomyces cerevisiae* to grow slowly or less strong, eventually leading to slow and incomplete alcoholic fermentation and low liquor yield. In actual production of alcohol and brewing, although raw materials contain rich nutrients, there still have some nutrients having insufficient content, such as nitrogen, magnesium, and phosphorus. The brewing materials also lacks some vitamins essential for the strong growth of the yeast, for example, the content of biotin is very low in molasses, therefore, extra biotin has to be added.

Major nutrient sources for *Saccharomyces cerevisiae* include a carbon source, a nitrogen source, inorganic salts, and vitamins.

The carbon source is rich in raw materials and thus does not need supplementing.

The nitrogen source constitutes proteins and nucleases in a yeast cell and serves as a nitrogen source for catabolites. The inorganic salts are indispensable substances for life activities of yeasts with main functions of: being a component of a cell; being an integral part of an enzyme reactive group or maintaining activities of an enzyme; adjusting osmotic pressure, pH, oxidation-reduction potential, etc; and being an energy source for an autotroph.

Main inorganic salts in a yeast cell include magnesium salts and phosphates, in which the magnesium salts often function as an activator for most of the enzymatic reactions. In a yeast, the content of magnesium salts (MgO) accounts for approx. 0.45% of its dry weight and 3.77 - 6.34% of the yeast ash content. Under normal circumstances, effective MgO content is insufficient in raw materials, and thus it needs to be supplemented. Phosphates account for approx. half (44.8% - 59.4%) of the yeast ash content. Phosphorus is a component element of nucleic acid, phosphatide, and many coenzymes, such as coenzyme 1, coenzyme 2, and coenzyme A. It involves in the phosphorylation process of carbohydrates to produce energy-rich phosphate (ATP) for energy storage and transmission. Meanwhile, the phosphates are important pH buffers.

Different types of vitamins are required for the growth of yeasts including biotin, pantothenic acid, inositol, and thiamine. Generally, except those mentioned above, other types of vitamins can be provided in raw materials for the growth of the yeasts.

Until now, there is not a solution in the art to combine a yeast extract, acid protease, and magnesium sulfate in a reasonable proportion to develop a nutrient composition for *Saccharomyces cerevisiae* with significant effects.

For example, although US20100184196 A1 and US20080187987 A1 relate to yeast extracts, neither of them discloses a nutrient composition for *Saccharomyces cerevisiae* that combines a yeast extract, acid protease, and magnesium sulfate.

Specifically, US20100184196 A1 discloses a composite yeast for high concentration alcohol fermentation. The composite yeast includes 40-70 parts by weight of the dried yeast, 15-35 parts by weight of a nitrogen source, 3-7 parts by weight of a phosphorous source, 10-15 parts by weight of a yeast gourmet, and 0.5-1.2 parts by weight of a bacteriostatic. In addition, the composite yeast includes 2.5-5 parts by weight of an inorganic salt selected from magnesium sulfate, zinc sulfate, or the mixture thereof.

In addition, US20080187987 A1 discloses a composite yeast for high concentration alcohol fermentation. The composite yeast includes 61-83 parts by weight of thermostable *Saccharomyces cerevisiae,* 5-30 parts by weight of acid protease, 3-10 parts by weight of phytase, 2-10 parts by weight of cellulose, 1-2 parts by weight of β-glucanase, and 1-2 parts by weight of pectinase.

Voigt Global Distribution Inc. (Voigt Global Distribution Inc.: "Difco Yeast Extract, Difco Yeast Nitrogen Base, Difco Yeast Nitrogen Base w/o Amino Acids, Difco Yeast Nitrogen Base w/o Amino Acids and Ammonium Sulfate", [Online], Retrieved from the internet: URL: http://www.voigtglobal.com/Anonymous/DIFCO_Yeast_Agar_Formulations.pdf [retrieved on 2007-08-29]) discloses a yeast extract. The extract includes nitrogen, phosphorus, amino acids, biotin, and inositol.

In addition, WO 92/20777 discloses a process of producing ethanol from raw materials that contain a high dry solids mash level. The process consists of liquefying the raw materials in the presence of an alpha-amylase to obtain liquefied mash; saccharifying the liquefied mash in the presence of a glucoamylase to obtain hydrolyzed starch and sugars; fermenting the hydrolyzed starch and sugars by yeast to obtain ethanol; and recovering ethanol.

The invention provides a highly effective nutrient composition for *Saccharomyces cerevisiae* comprising a yeast extract, acid protease, and magnesium sulfate according to a scientific proportion, in which the yeast extract comprises nitrogen, phosphorus, amino acids, biotin, pantothenic acid, inositol, and thiamine. It solves the disadvantages in actual production of alcohol and brewing, for example, slow or incomplete alcoholic fermentation and low liquor yield caused by insufficient nutrients in raw materials for the growth of yeasts and manual supplementation of nutrients, which is single and incomplete, or the addition amount is either too much or too little. The nutrient composition for *Saccharomyces cerevisiae* provided by the invention can reduce the fermentation period by 5 - 15 hrs and increase alcohol by volume (ABV) by 1-2% (v/v).

Yeast extract is produced using food yeasts with rich proteins as the raw material and by modern biotechnology, such as autolysis and refinement. It is rich in nitrogen, phosphorus, amino acid, biotin, pantothenic acid, inositol, and thiamine. Yeast extract, such as yeast extract powder FM818, yeast autolytic powder FM901, and FM801, can be bought from Angel Yeast Co., Ltd.

Acid protease is a type of solid and gray powdered enzyme with activity at approx. 50,000 u/g and it is easily accessible from companies producing enzyme preparation. In the invention, different types of acid proteases can be used.

Magnesium sulfate is a type of solid and white chemical material with fine granules and it is easily accessible from companies selling chemical materials.

The nutrient composition for *Saccharomyces cerevisiae* provided by the invention comprises:
a yeast extract: 62 - 98 weight parts;
an acid protease: 1.5 - 20 weight parts; and
magnesium sulfate: 0.1 - 2.5 weight parts.

Preferably, the nutrient composition for *Saccharomyces cerevisiae* provided by the invention comprises:
the yeast extract: 85 - 95 weight parts;
the acid protease: 4 - 13 weight parts; and
magnesium sulfate: 0.5 - 1.5 weight parts.

More preferably, the nutrient composition for *Saccharomyces cerevisiae* provided by the invention comprises:
the yeast extract: 90 weight parts;
the acid protease: 9 weight parts; and
magnesium sulfate: 1.0 weight part.

Amount of the acid protease used in the invention is calculated based on enzyme activity unit of 50,000 u/g. If the activity unit thereof is not 50,000 u/g, the amount of the acid protease must be changed accordingly.

The nutrient composition provided by the invention further comprises zinc sulfate, ammonium dibasic phosphate, and calcium chloride, which make the nutrient composition for *Saccharomyces cerevisiae* have more complete nutrients, more stable performance, and wider applicable materials.

The nutrient composition provided by the invention are applicable to the alcoholic fermentation of not only starch materials, such as corn, cassava, wheat, and rice, but also saccharine materials, such as molasses, sucrose, and sugar sorghum.

The invention provides a method of alcoholic fermentation. The method comprises adding a nutrient composition to a fermentation substrate, the fermentation substrate being corn, cassava, wheat, rice, molasses, sucrose, or sugar sorghum, and the nutrient composition comprising: a) 90 parts by weight of a yeast extract; b) 9 parts by weight of an acid protease having an enzyme activity of about 50,000 U/g; and c) 1.0 parts by weight of magnesium sulfate.. The addition proportion is a ratio of the mass of the nutrient composition added to the mass of raw materials to be fermented. Generally, the amount of the nutrient composition is 0.1 - 1% (w/w), preferably 0.4 - 0.6% (w/w), and more preferably 0.5% (w/w) of the fermentation substrate.

The nutrient composition provided by the invention can stimulate strong growth and reproduction of *Saccharomyces cerevisiae* in various brewing materials, thereby speeding up alcoholic fermentation, shortening alcoholic fermentation period, and increasing liquor yield. The nutrient composition can shorten the alcoholic fermentation period by 5-15 hrs thereby enhancing the equipment utilization, and increase final ABV by 1 - 2% (v-v) thereby saving the production costs of alcohol per ton by RMB 50 - 200.

For further illustrating the invention, experiments detailing a nutrient composition for *Saccharomyces cerevisiae* and a usage method thereof are described below. It should be noted that the following examples are intended to describe and not to limit the invention.

All raw materials and instruments used in the examples are shown in table 1 :

**Table 1**

| Raw Materials | Origin/Manufacturers | Model |
|---|---|---|
| Thermostable amylase | Novozymes (China) Investment Co Ltd. | 20,000 u/mL |
| Glucoamylase | Novozymes (China) Investment Co Ltd. | 100,000 u/mL |
| Thermostable *Saccharomyces cerevisiae* | Angel Yeast Co., Ltd. | Dry yeast with high-temperature resistance and high activity from Angel Yeast Co., Ltd. |
| Alcoholmeter | Qingxian Measuring Glass Apparatus Factory | 10-20%vol |

Corn is used as a raw material in the following embodiments for brewing using conventional fermentation methods. In the process of brewing, a nutrient is added to evaluate its influence on the alcoholic fermentation process.

### Fermentation Procedures

1. Selection of raw materials: sieve the corn powder with a 1.5 mm mesh sieve.
2. Moisture treatment: add 240 g of water with a temperature of 60 - 70°C into 100 g of the corn powder obtained from the step 1 based on a material-water ratio of 1: 2.4 and moistens the material for 30 mins.
3. Liquefaction: add a thermostable amylase for every 10 u/g corn powder (or 10-20 u/g corn powder) and liquefy them under 96°C (95 - 97°C) for 1.5 hrs (1.5 - 2 hrs), afterwards, cool them down to 32°C and adjust pH = 4.2 (pH 4.2 - 4.5) with 50% dilute sulfuric acid.
4. Add a nutrient for *Saccharomyces cerevisiae* of the invention.
5. Liquor fermentation: add glucoamylase for every 180 u/g (150 - 200 u/g) corn powder and dry yeast with high-temperature resistance and high activity from Angel Yeast Co., Ltd. based on 0.2% (w/w) corn powder, and then ferment under 33°C for 72 hrs.
6. Measure the weight loss of carbon dioxide once per 24 hrs.
7. Distillation: make liquor by distillation with conventional brewing processes i.e. measure 100 mL fermented liquor using a graduated cylinder and put it into a 1000 mL distillation flask and then add 1 - 2 drops of anti-foaming agent (e.g. salad oil) for fermentation. Collect the distillates using a 100 mL measuring flask (with an ice water bath). When the distillates reach approx. 95 mL, stop distillation and take the measuring flask down and when the temperature reaches the room temperature, dilute the solution to 100 mL.
8. Measurement of ABV: put the diluted distillates into a clean and dry graduated cylinder (100 mL), stand for a few minutes until air bubbles disappear from the liquor and then transfer to a clean and precise alcoholmeter and slightly press once. Stand for a while, observe the scale value at the tangent point of the lowest arc surface of the liquid at eye level and measure the temperature of the liquid, convert it to the ABV under 20°C according to Alcoholmeter Temperature and Concentration Conversion Table.

### Example 1

### Influence of Yeast Extract on Alcoholic Fermentation of Corn

The above-mentioned fermentation procedures were carried out for alcoholic fermentation of corn, and the nutrient added in step 4 was a yeast extract which comprises nitrogen, phosphorus, amino acids, biotin, pantothenic acid, inositol, and thiamine (yeast extract powder FM818 produced and provided by Angel Yeast Co., Ltd.) with amount (based on weight percentage of fermentation substrate) of 0, 0.05%, 0.1%, 0.25%, and 0.50%, respectively. The chemical properties of the yeast extract are listed in table 2.

**Table 2**

| | |
|---|---|
| Appearance | Yellow or brown, powdery |
| Smell | Smell of yeast extract |
| Total nitrogen (dry weight) | ≥ 10.0% |
| Ammonia nitrogen (dry weight) | ≥ 4.0% |
| Moisture | ≤ 6.0% |
| Sodium chloride | ≤ 2.0% |
| Ash | ≤ 15.0% |
| pH (2% aqueous solution) | 5.3-7.2 |
| Lead | ≤ 2.0 mg/kg |
| Total arsenic | ≤ 2.0 mg/kg |
| Total number of colonies | ≤ 5×10⁴ cfu/g |
| *Coliform* | ≤ 0.3 MPN/g |
| *Salmonella* | None |
| *Staphylococcus aureus* | None |
| *Shigella* | None |

The weight loss of carbon dioxide (g) was measured every 24 hrs. When the reaction was over, the final ABV (v/v) was measured. The test results are shown in table 3.

**Table 3 Influence of Yeast Extract on Alcoholic Fermentation of Corn**

| Yeast extract (%) | 0.00 | 0.05 | 0.1 | 0.25 | 0.50 |
|---|---|---|---|---|---|
| 0-24hrs weight loss of carbon dioxide (g) | 11.94 | 11.72 | 11.37 | 13.35 | 19.07 |
| 24-48hrs weight loss of carbon dioxide (g) | 12.40 | 11.98 | 12.65 | 11.66 | 12.39 |
| 48-72hrs weight loss of carbon dioxide (g) | 9.73 | 10.73 | 10.69 | 9.78 | 3.89 |
| Total weight loss of carbon dioxide (g) | 34.0 7 | 34.4 3 | 34.7 1 | 34.7 9 | 35.3 5 |
| ABV (v/v) | 12.9 0 | 13.2 0 | 13.4 0 | 13.4 0 | 13.7 0 |

As shown in table 3, the yeast extract has a big influence on alcoholic fermentation of corn, the ABV is increased, and based on weight loss of carbon dioxide, and the fermentation speed is also enhanced significantly.

### Example 2

### Influence of Acid Protease on Alcoholic Fermentation of Corn

The above-mentioned fermentation procedures were carried out for alcoholic fermentation of corn, and the nutrient added in step 4 was an acid protease (provided by Novozymes (China) Investment Co Ltd.; enzyme activity 50,000 u/g) with amount (based on weight percentage of fermentation substrate) of 0, 0.01%, 0.03%, 0.05%, and 0.07%, respectively. The weight loss of carbon dioxide (g) was measured every 24 hrs. When the reaction was over, the final ABV (v/v) was measured. The test results are shown in table 4.

**Table 4**

| Acid protease (%) | 0.00 | 0.01 | 0.03 | 0.05 | 0.07 |
|---|---|---|---|---|---|
| 0-24 hrs weight loss of carbon dioxide (g) | 11.85 | 20.10 | 21.85 | 19.86 | 15.07 |
| 24-48 hrs weight loss of carbon dioxide (g) | 12.78 | 12.42 | 12.13 | 13.75 | 19.71 |
| 48-72 hrs weight loss of carbon dioxide (g) | 9.38 | 2.12 | 1.37 | 1.86 | 0.29 |
| Total weight loss of carbon dioxide (g) | 34.01 | 34.64 | 35.35 | 35.47 | 35.07 |
| ABV | 12.90 | 13.50 | 13.80 | 13.80 | 13.70 |

As shown in table 4, the acid protease has a big influence on alcoholic fermentation of corn, the ABV is increased accordingly, and based on weight loss of carbon dioxide, and the fermentation speed is also enhanced significantly.

### Example 3

### Influence of Magnesium Sulfate on Alcoholic Fermentation of Corn

The above-mentioned fermentation procedures were carried out for alcoholic fermentation of corn, and the nutrient added in step 4 was magnesium sulfate (provided by Yichang Xilong Chemical Store) with amount (based on weight percentage of fermentation substrate) of 0, 0.001%, 0.003%, 0.005%, and 0.007%, respectively. The weight loss of carbon dioxide (g) was measured every 24 hrs. When the reaction was over, the final ABV (v/v) was measured. The test results are shown in table 5.

**Table 5**

| Magnesium sulfate (%) | 0 | 0.001 | 0.003 | 0.005 | 0.007 |
|---|---|---|---|---|---|
| 0-24hrs weight loss of carbon dioxide (g) | 11.83 | 12.96 | 13.06 | 12.85 | 12.23 |
| 24-48hrs weight loss of carbon dioxide (g) | 13.64 | 18.81 | 18.37 | 18.46 | 18.89 |
| 48-72hrs weight loss of carbon dioxide (g) | 8.58 | 3.11 | 3.39 | 3.51 | 3.69 |
| Total weight loss of carbon dioxide (g) | 34.05 | 34.88 | 34.82 | 34.82 | 34.81 |
| ABV (v/v) | 12.90 | 13.6 | 13.5 | 13.6 | 13.6 |

As shown in table 5, the magnesium sulfate has a big influence on alcoholic fermentation of corn, the ABV is increased, and based on weight loss of carbon dioxide, and the fermentation speed is also enhanced significantly.

### Example 4

### Influence of Yeast Extract, Acid Protease, and Magnesium Sulfate on Alcoholic Fermentation of Corn

The above-mentioned fermentation procedures were carried out for alcoholic fermentation of corn, and the nutrient added in step 4 was a yeast extract which comprises nitrogen, phosphorus, amino acids, biotin, pantothenic acid, inositol, and thiamine, acid protease, magnesium sulfate, or a mixture thereof, with amount of 0.5%. The weight loss of carbon dioxide (g) was measured every 24 hrs. When the reaction was over, the final ABV (v/v) was measured. The test results are shown in table 6.

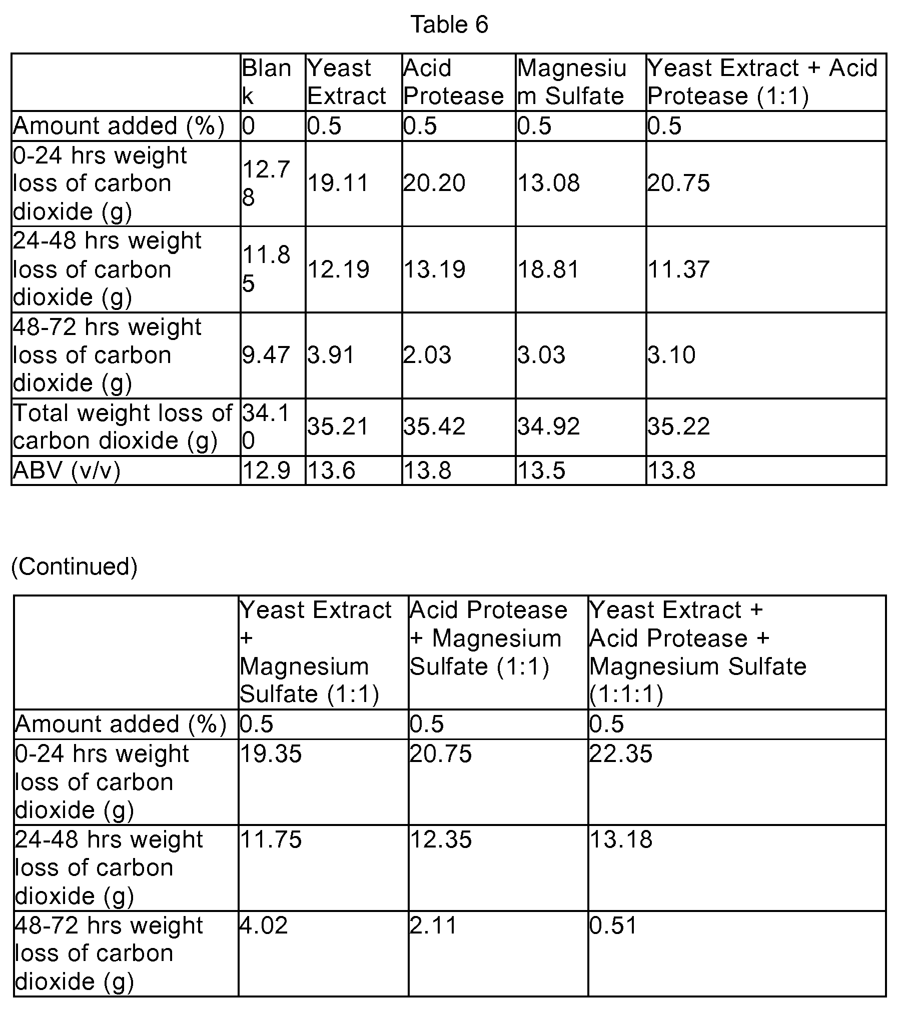

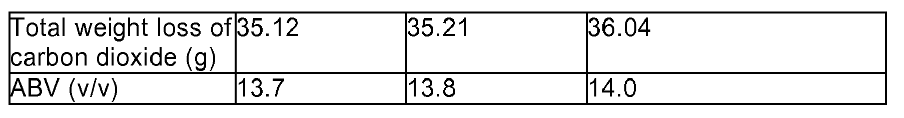

As shown in table 6, the combination of the yeast extract, acid protease, and magnesium sulfate the magnesium sulfate has bigger influence on alcoholic fermentation of corn. The fermentation speed and ABV are increased significantly. The combination of those three substances has much more noticeable effect than that produced by an individual one.

### Example 5

### Influence of Nutrient composition for Saccharomyces cerevisiae on Alcoholic Fermentation of Corn

The above-mentioned fermentation procedures were carried out for alcoholic fermentation of corn, and the nutrient added in step 4 was listed in table 7. The percentage of each nutrient to be added and the test results are shown in table 7.

**Table 7**

| | | Blank | Test 1 | Test 2 | Test 3 | Test 4 | Test 5 | Test 6 |
|---|---|---|---|---|---|---|---|---|
| Formula | Yeast Extract | 0 | 90 | 98 | 95 | 85 | 70 | 62 |
| | Acid Protease | 0 | 9 | 1.5 | 4 | 20 | 13 | 3 |
| | Magnesium Sulfate | 0 | 1.0 | 0.5 | 1.5 | 0.1 | 2.5 | 2.0 |
| 0-12hrs weight loss of carbon dioxide (g) | | 5.1 | 10.07 | 9.22 | 10.04 | 10.02 | 9.89 | 9.55 |
| 12-24hrs weight loss of carbon dioxide (g) | | 6.65 | 12.38 | 11.45 | 11.93 | 11.62 | 11.60 | 11.53 |
| 24-36hrs weight loss of carbon dioxide (g) | | 6.85 | 9.05 | 8.62 | 8.80 | 8.95 | 8.97 | 8.58 |
| 36-48hrs weight loss of carbon dioxide (g) | | 6.4 | 4.82 | 5.96 | 4.92 | 4.96 | 4.93 | 5.69 |
| 48-64hrs weight loss of carbon dioxide (g) | | 5.72 | 0.26 | 1.02 | 0.79 | 0.86 | 0.82 | 0.88 |
| 64-72hrs weight loss of carbon dioxide (g) | | 3.3 | 0 | 0 | 0 | 0 | 0 | 0 |
| Total weight loss of carbon dioxide (g) | | 34.02 | 36.58 | 36.27 | 36.48 | 36.41 | 36.21 | 36.23 |
| ABV (v/v) | | 12.8 | 14.5 | 14.2 | 14.4 | 14.3 | 14.2 | 14. 2 |

As shown in table 7, the nutrient composition for *Saccharomyces cerevisiae* has significant influence on the alcoholic fermentation of corn. The fermentation speed is increased significantly, fermentation period is reduced by more than 12 hours, ABV is increased by 1.7(v/v), and the liquor yield (ABV) is largely increased. The above tests show that the preferred percentage of the nutrient composition for *Saccharomyces cerevisiae* by weight is that yeast extract 90, acid protease 9, and magnesium sulfate 1.0.

### Example 6

### Influence of Added Proportion of Nutrient composition for Saccharomyces cerevisiae on Alcoholic Fermentation of Corn

The above-mentioned fermentation procedures were carried out for alcoholic fermentation of corn, and the nutrient added in step 4 was the nutrient composition for *Saccharomyces cerevisiaes* with the proportion listed in test 1 of table 7. The addition proportion of the nutrient composition for *Saccharomyces cerevisiaes,* which was a weight percent of the fermentation substrate, is listed in table 8. When the reaction was over, final ABV (v/v) was measured. The test results are shown in table 8.

**Table 8**

| | | | | | | | | | | | |
|---|---|---|---|---|---|---|---|---|---|---|---|
| Nutrient composition for *Saccharomyces cerevisiae* (%) | 0 | 0.1 | 0.2 | 0.3 | 0.4 | 0.5 | 0.6 | 0.7 | 0.8 | 0.9 | 1 |
| ABV (v/v) | 12.8 | 13.9 | 13.9 | 14.2 | 14.4 | 14.5 | 14.5 | 14.5 | 14.5 | 14.5 | 14.5 |

As shown in table 8, the ABV is increased significantly when the nutrient composition for *Saccharomyces cerevisiaes* are added with the amount of 0.1%, 0.2%, 0.3%, 0.4%, 0.5%, 0.6%, 0.7%, 0.8%, 0.9%, and 1% of the fermentation substrate by weight. In terms of fermentation results and use costs, 0.4 - 0.6% of addition proportion is more appropriate and 0.5% (based on weight ratio of fermentation substrate) is more preferred.

### Example 7

### Influence of Nutrient composition for Saccharomyces cerevisiae Added with Cofactor (e.g. zinc sulfate) on Alcoholic Fermentation of Corn

The above-mentioned fermentation procedures were carried out for alcoholic fermentation of corn, and the nutrient added in step 4 was the nutrient composition for *Saccharomyces cerevisiaes* with the proportion listed in test 1 of table 7, or zinc sulfate was further added to the nutrients listed in test 1 of table 7 accounting for 0.5% of the total amount. Each of the above two types of nutrients accounts for 0.5% of the added fermentation substrate. When the reaction was over, the final ABV (v/v) was measured. The test results are shown in table 9.

**Table 9**

| | Blank | Nutrient composition for *Saccharomyces cerevisiae* (incl. 0.5% zinc sulfate) | Nutrient composition for *Saccharomyces cerevisiae* (excl. zinc sulfate) |
|---|---|---|---|
| ABV (v/v) | 12.8 | 14.6 | 14.5 |

The fermentation results show that the ABV is increased by 0.1 (v/v) in alcoholic fermentation of corn with the nutrient composition added with the cofactor zinc sulfate compared with the one without the zinc sulfate.

The main purpose of adding a cofactor is to make the nutrient composition for *Saccharomyces cerevisiae* have complete nutrients, stable performance, and wide applicable materials. It is applicable to the alcoholic fermentation of not only starch materials, such as corn, cassava, wheat, and rice, but also saccharine materials, such as molasses, sucrose, and sugar sorghum.

## Claims

1. A method of alcoholic fermentation, the method comprising adding a nutrient composition for *Saccharomyces cerevisiae* to a fermentation substrate, the fermentation substrate being corn, cassava, wheat, rice, molasses, sucrose, or sugar sorghum, an amount of the nutrient composition being 0.1 - 1 wt. % of the fermentation substrate, and the nutrient composition comprising:
a) a yeast extract: 90 parts by weight;
b) an acid protease having an enzyme activity of about 50,000 U/g: 9 parts by weight; and
c) magnesium sulfate: 1.0 part by weight.

2. The method of claim 1, **characterized in that** the amount of the nutrient composition is 0.4 - 0.6 wt. % of the fermentation substrate.

3. The method of claim 1 or 2, **characterized in that** the amount of the nutrient composition is 0.5 wt. % of the fermentation substrate.

## Patentansprüche

1. Verfahren zur alkoholischen Fermentation, wobei das Verfahren die Zugabe einer Nährstoffzusammensetzung für *Saccharomyces cerevisiae* zu einem Fermentationssubstrat umfasst, wobei das Fermentationssubstrat Mais, Maniok, Weizen, Reis, Melasse, Saccharose oder Zuckerhirse ist, wobei eine Menge der Nährstoffzusammensetzung 0,1 bis 1 Gew.-% des Fermentationssubstrats beträgt und die Nährstoffzusammensetzung umfasst:
a) einen Hefeextrakt: 90 Gewichtsteile;
b) eine saure Protease mit einer Enzymaktivität von ungefähr 50.000 U/g: 9 Gewichtsteile; und
c) Magnesiumsulfat: 1,0 Gewichtsteil.

2. Verfahren nach Anspruch 1, **dadurch gekennzeichnet, dass** die Menge der Nährstoffzusammensetzung 0,4 bis 0,6 Gew.-% des Fermentationssubstrats beträgt.

3. Verfahren nach Anspruch 1 oder 2, **dadurch gekennzeichnet, dass** die Menge der Nährstoffzusammensetzung 0.5 Gew.-% des Fermentationssubstrats beträgt.

## Revendications

1. Procédé de fermentation alcoolique, le procédé comprenant l'ajout d'une composition nutritive pour *Saccharomyces cerevisiae* à un substrat de fermentation, le substrat de fermentation étant du maïs, du manioc, du blé, du riz, de la mélasse, du saccharose, ou du sorgho à sucre, une quantité de la composition nutritive étant de 0,1 à 1 % en poids du substrat de fermentation, et la composition nutritive comprenant :
a) un extrait de levure : 90 parties en poids ;
b) une protéase acide ayant une activité enzymatique d'environ 50 000 U/g : 9 parties en poids ; et
c) du sulfate de magnésium : 1,0 partie en poids.

2. Procédé selon la revendication 1, **caractérisé en ce que** la quantité de la composition nutritive est de 0,4 à 0,6 % en poids du substrat de fermentation.

3. Procédé selon la revendication 1 ou 2, **caractérisé en ce que** la quantité de la composition nutritive est de 0,5 % en poids du substrat de fermentation.
